# EUROPEAN PATENT APPLICATION

(11) **EP 3 050 527 A1**
(43) Date of publication of application: **03.08.2016**
(21) Application number: 14847518.9
(22) Date of filing: 25.09.2014
(51) Int. Cl.: A61B 18/00

(54) **TREATMENT TOOL AND TREATMENT SYSTEM**

(30) Priority: 27.09.2013 US 201361883520 P; 26.12.2013 WO PCT/JP2013/084925
(71) Applicant: OLYMPUS CORPORATION, Shibuya-ku Tokyo 151-0072 (JP)
(72) Inventor: YACHI, Chie, Tokyo, 151-0072 (JP)
(74) Representative: Gunzelmann, Rainer
(86) International application number: PCT/JP2014/075461
(87) International publication number: WO 2015/046349

(57) **Abstract**

A treatment device for use in treating a biological tissue, includes: an ultrasonic transducer unit; a probe; an actuator that moves the ultrasonic transducer unit and the probe integrally along the longitudinal axis at a stroke larger than an amplitude of the ultrasonic vibration to be transmitted to the probe; an inner sheath that covers an outer peripheral surface of the probe and move with the probe accordance with a movement of the actuator; a first sealing member that seals between an inner peripheral surface of the inner sheath and the outer peripheral surface of the probe and that prevent penetration of a liquid to a proximal side of an outer peripheral surface of the probe along the longitudinal axis from an seal position; an outer sheath that is disposed on an outer peripheral surface of the inner sheath; and a second sealing member that seals between the outer peripheral surface of the inner sheath and an inner peripheral surface of the outer sheath and that prevents the penetration of the liquid to an out peripheral surface of the probe and the ultrasonic transducer unit.

## Description

### Technical Field

This invention relates to a treatment device for use in treating a biological tissue, and a treatment system having the treatment device.

### Background Art

For example, in a publication of Jpn. PCT National Publication No. 2013-519434, an ultrasonic treatment device is disclosed. The ultrasonic treatment device includes a blade that performs an ultrasonic vibration, a hollow sheath that covers the blade and is hollow, and a motor that allows the blade to perform a translational motion to the hollow sheath in an axial direction of the blade. Further, the ultrasonic treatment device enables an operator to select an ultrasonic mode in which the blade is ultrasonically vibrated to treat a hard biological tissue such as a bone, or a translational motion mode in which the blade is translationally moved by the motor to treat a hard biological tissue such as a bone.

### Citation List

### Patent Literature

Patent Literature 1: Jpn. PCT National Publication No. 2013-519434

### Summary of Invention

In an ultrasonic treatment device of a publication of Jpn. PCT National Publication No. 2013-519434, a sealing member is interposed between a hollow sheath and a blade to prevent penetration of a liquid. However, in the ultrasonic treatment device of the publication of Jpn. PCT National Publication No. 2013-519434, the blade is translationally moved to the hollow sheath in an axial direction of the blade, and hence, between the sealing member and the hollow sheath or between the sealing member and the blade, a gap is easily made, through which the liquid might penetrate. As a result, there is the fear that an ultrasonic vibration of the blade is obstructed.

Thus, an object of this invention is to provide a treatment device and a treatment system which is capable of moving a probe to a sheath in an axial direction of the probe, and preventing an ultrasonic vibration of the probe from being obstructed.

According to one aspect of the present invention, a treatment device for use in treating a biological tissue, includes: a holding portion to be held by a user; an ultrasonic transducer unit that generates an ultrasonic vibration in accordance with a supply of a power; a probe which defines a longitudinal axis, is configured to transmit the ultrasonic vibration generated by the ultrasonic transducer unit, and has a treatment portion configured to treat the biological tissue by an action of the ultrasonic vibration transmitted to a distal end portion of the probe; an inner sheath that covers an outer peripheral surface of the probe; a first sealing member that is interposed between an inner peripheral surface of the inner sheath and the outer peripheral surface of the probe, and comes in contact closely with the inner peripheral surface of the inner sheath and the outer peripheral surface of the probe to prevent penetration of a liquid; an outer sheath that covers an outer peripheral surface of the inner sheath; a second sealing member that is interposed between the outer peripheral surface of the inner sheath and an inner peripheral surface of the outer sheath, and comes in contact closely with the outer peripheral surface of the inner sheath and the inner peripheral surface of the outer sheath to prevent the penetration of the liquid; and an actuator that moves the ultrasonic transducer unit, the probe and the inner sheath integrally to the outer sheath along the longitudinal axis at a stroke larger than an amplitude of the ultrasonic vibration to be transmitted to the treatment portion of the probe.

### Advantageous Effects of Invention

According to the present invention, there can be provided a treatment device and a treatment system which is capable of moving a probe to a sheath in an axial direction of the probe and preventing an ultrasonic vibration of the probe from being obstructed.

### Brief Description of Drawings

FIG. 1 is a schematic view showing a treatment system according to a first embodiment;
FIG. 2 is a schematic partial vertical cross-sectional view of a treatment device of the treatment system according to the first embodiment;
FIG. 3A is a schematic partial vertical cross-sectional view showing a portion from the vicinity of a rotary operating knob to a distal end side in the treatment device of the treatment system according to the first embodiment;
FIG. 3B is a schematic lateral cross-sectional view taken along the 3B-3B line in FIG. 3A;
FIG. 4 is a schematic block diagram showing the treatment system according to the first embodiment;
FIG. 5 is a schematic view showing that a biological tissue is moved to be cut in a blade surface region of a probe of the treatment device of the treatment system according to the first embodiment;
FIG. 6 is a schematic view showing a treatment system according to a second embodiment;
FIG. 7 is a partial vertical cross-sectional view showing a schematic structure of a treatment device of the treatment system according to the second embodiment;
FIG. 8A is a schematic partial vertical cross-sectional view showing a treatment system according to a third embodiment;
FIG. 8B is a schematic enlarged view of a position shown by reference sign 8B in a treatment device shown in FIG. 8A of the treatment system according to the third embodiment;
FIG. 9 is a schematic partial vertical cross-sectional view showing a treatment system according to a fourth embodiment;
FIG. 10A is a schematic partial vertical cross-sectional view showing a treatment device according to a fifth embodiment;
FIG. 10B is a schematic partial vertical cross-sectional view showing the treatment device according to the fifth embodiment;
FIG. 10C is a schematic partial vertical cross-sectional view of the treatment device according to the fifth embodiment taken along the 10C-10C line in FIG. 10A and FIG. 10B;
FIG. 10D is a schematic enlarged view of the treatment device according to the fifth embodiment at a position shown by reference sign 10D in FIG. 10A;
FIG. 10E is a schematic enlarged view of the treatment device according to the fifth embodiment at a position shown by reference sign 10E in FIG. 10A;
FIG. 11A is a schematic perspective view showing a rake type of treatment portion of a probe which is usable in the first to fifth embodiments;
FIG, 11B is a schematic perspective view showing a curette type of treatment portion of the probe which is usable in the first to fifth embodiments; and
FIG. 11C is a schematic perspective view showing a blade type of treatment portion of the probe which is usable in the first to fifth embodiments.

### Description of Embodiments

Hereinafter, embodiments of this invention will be described with reference to the drawings.

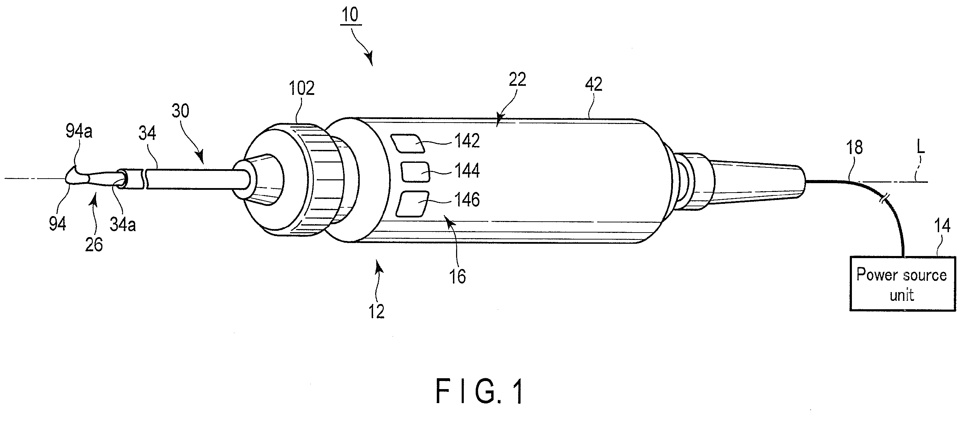

A first embodiment is described with reference to FIG. 1 to FIG. 5.

As shown in FIG. 1, a treatment system 10 according to this embodiment includes a treatment device 12 for use in treating a biological tissue, a power source unit (a controller) 14, and a switch portion 16 such as a foot switch or a hand switch. The treatment device 12 is connected to the power source unit 14 via a cable 18. Through the cable 18, the switch portion 16 transmits a signal to the power source unit 14 and receives a signal from the power source unit, and supplies a power controlled by the power source unit 14 to an after-mentioned ultrasonic transducer 82 of the treatment device 12. It is to be noted that, in the present embodiment, the cable 18 is extended from a proximal end of the treatment device 12, and an unshown connector of a distal end of the cable 18 is attachable to and detachable from the power source unit 14.

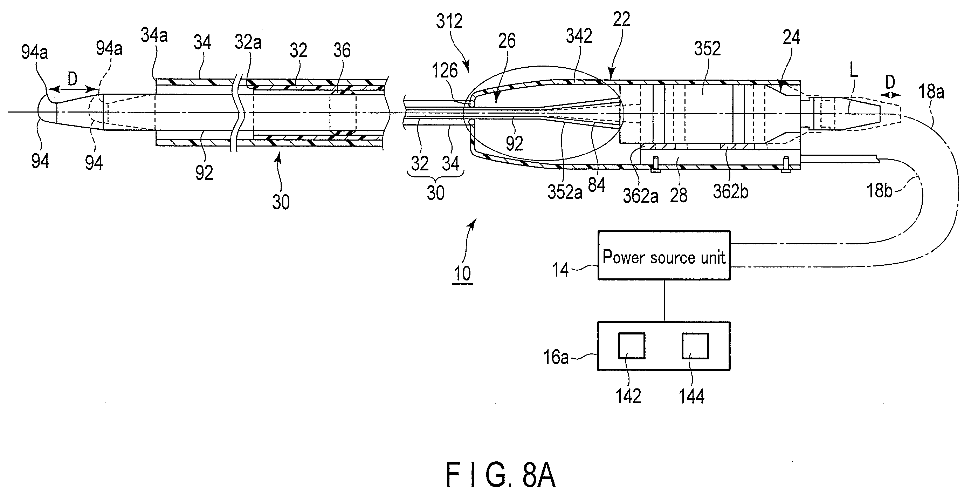

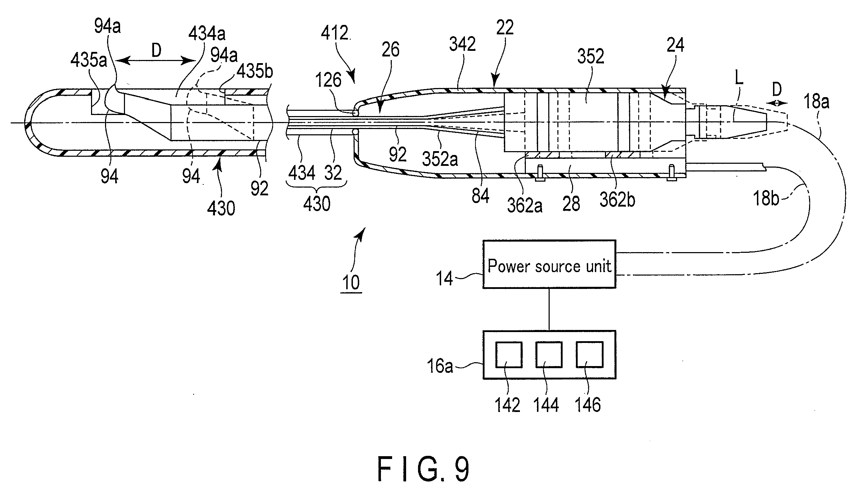

Additionally, in the present embodiment, as the switch portion 16, the hand switch disposed in the treatment device 12 itself is used, but as described later in a third embodiment (see FIG. 8A) and a fourth embodiment (see FIG. 9), a foot switch 16a to be connected to the power source unit 14 is preferably used. The foot switch 16a is also attachable to and detachable from the power source unit 14 in the same manner as in the treatment device 12.

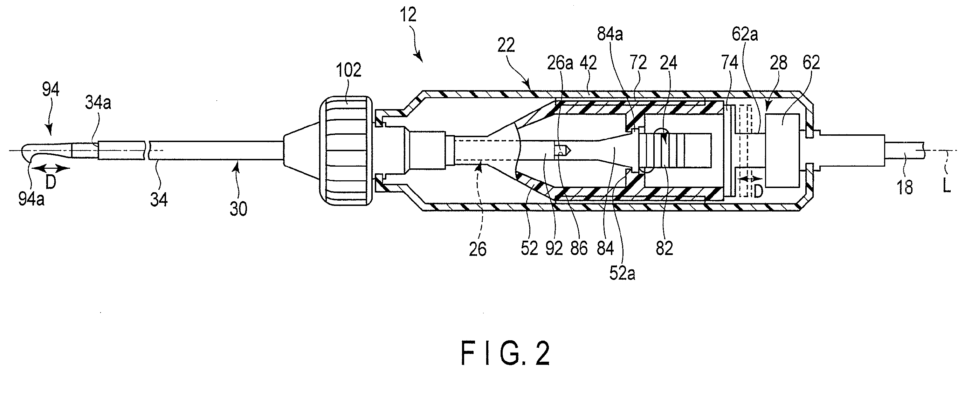

As shown in FIG. 2, the treatment device 12 includes a holding portion 22 to be held by a user, an ultrasonic transducer unit 24, a probe 26, and an actuator 28.

In this embodiment, the holding portion 22 has a central axis on a longitudinal axis L defined by the probe 26. The ultrasonic transducer unit 24, the probe 26 and a tubular body 30 are arranged to the holding portion 22 at the center of the longitudinal axis L.

The holding portion 22 includes a tubular outer case 42 preferably having, for example, electric insulating properties. On an inner side of the outer case 42, there are arranged a tubular inner case (a cover for the transducer unit) 52 that supports the ultrasonic transducer unit 24 on the inner side of the inner case 52, and the actuator 28 that moves the inner case 52 along the longitudinal axis L. In this embodiment, the outer case 42 and the inner case 52 are present at concentric positions at the center of the longitudinal axis L, and the actuator 28 is supported at a proximal end of the outer case 42.

The actuator 28 can move the ultrasonic transducer unit 24 and the probe 26 integrally to the holding portion 22 along the longitudinal axis L at a stroke larger than an amplitude of an ultrasonic vibration to be transmitted to a treatment portion 94 of the probe 26. As the actuator 28, a linear motor 62 having a linear actuating rod 62a is used in this embodiment. In the linear motor 62, the linear actuating rod 62a expands and contracts along the longitudinal axis L, whereby the inner case 52 can be moved to the outer case 42 along the longitudinal axis L. In other words, the inner case 52 is moved by the actuator 28, whereby the ultrasonic transducer unit 24 and the probe 26 are moved by the actuator 28 at the stroke larger than the amplitude (e.g., about several tens µm) of the ultrasonic vibration to be transmitted to the treatment portion 94 of the probe 26 along the longitudinal axis L of the probe 26. A stroke of the linear actuating rod 62a is sufficiently larger than the amplitude of the ultrasonic vibration to be transmitted to the treatment portion 94 of the probe 26. For example, a maximum stroke of the linear actuating rod 62a can be from about 10 mm to 20 mm, and can suitably be set in accordance with a performance of the actuator 28 itself and by an after-mentioned setting section 134. It is to be noted that a minimum stroke of the linear actuating rod 62a can be set by the setting section 134, but is larger than the amplitude of the ultrasonic vibration to be transmitted to the treatment portion 94 of the probe 26.

As the actuator 28 according to this embodiment, needless to say, a ball screw may be combined with a rotary shaft of a rotary motor to directly operate the linear actuating rod 62a, though it is not shown in the drawing. That is, the actuator 28 may only move the ultrasonic transducer unit 24 and the probe 26 integrally to the holding portion 22 along the longitudinal axis L of the probe 26. In this case, the actuator 28 moves the ultrasonic transducer unit 24 and the probe 26 integrally at the stroke larger than the amplitude of the ultrasonic vibration.

A first bearing 72 is interposed between an inner peripheral surface of the outer case 42 and an outer peripheral surface of the inner case 52. By the first bearing 72, the inner case 52 is rotatable to the outer case 42 in a periaxial direction of the longitudinal axis L, and the inner case 52 is rotatable to the outer case 42 in an axial direction along the longitudinal axis L. It is to be noted that, when the inner case 52 does not have to be rotated to the outer case 42 in the periaxial direction of the longitudinal axis L, the first bearing 72 may only have a function of moving the inner case 52 to the outer case 42 in the axial direction along the longitudinal axis L.

The actuator 28 is fixed to the inner side of the outer case 42 and a proximal end of the inner case 52. Between the actuator 28 and the outer case 42, a second bearing 74 such as a ball bearing is interposed, and the inner case 52 is rotatably supported to the actuator 28. It is to be noted that, when the inner case 52 does not have to be rotated to the actuator 28, the second bearing 74 does not have to be disposed.

The transducer unit 24 and the probe 26 are supported in the inner case 52.

The transducer unit 24 includes the ultrasonic transducer 82 that generates the ultrasonic vibration by suitably supplying the power from the power source unit 14 shown in FIG. 1, and a conical horn 84 that enlarges the amplitude of the ultrasonic vibration generated in the ultrasonic transducer 82. That is, the transducer unit 24 generates the ultrasonic vibration in accordance with the supply of the power.

As the ultrasonic transducer 82, for example, a BLT type is used. The horn 84 is attached to an external thread 26a of a proximal end of the probe 26 by a connecting screw (an internal thread) 86. The horn 84 includes an outer flange 84a projected outwardly in a radial direction to the longitudinal axis L of the horn 84. The outer flange 84a is present at a node position of the ultrasonic vibration when the vibration is transmitted from the ultrasonic transducer 82.

In the inner case 52, an inner flange 52a projected inwardly from an inner peripheral surface in the radial direction is formed. The outer flange 84a of the horn 84 engages with the inner flange 52a of the inner case 52, whereby the transducer unit 24 and the probe 26 are supported in the inner case 52. That is, the inner case 52 is interposed between the holding portion 22 and the ultrasonic transducer unit 24, to support the ultrasonic transducer unit 24 at the node position of the ultrasonic vibration.

It is to be noted that, in this embodiment, there is described an example where the ultrasonic transducer unit 24 is supported in the inner case 52, but it is also preferable that the ultrasonic transducer unit 24 is attachable to and detachable from the inner case 52.

The probe 26 shown in FIG. 1 and FIG. 2 is designed so that an overall length of the probe is an integer multiple of a half wavelength of the ultrasonic vibration. The probe 26 includes a rod-like probe main body 92 made of a metal such as a titanium alloy material, and the treatment portion 94 disposed on a distal end side of the probe main body 92. Amplitude of the ultrasonic vibration generated by the ultrasonic transducer 82 is enlarged by the horn 84 and the enlarged amplitude is transmitted to the treatment portion 94 through the probe main body 92. The probe 26 is capable of transmitting the ultrasonic vibration from the ultrasonic transducer unit 24, and treating the biological tissue by an action of the ultrasonic vibration transmitted to the treatment portion 94 of a distal end portion of the probe. The treatment portion 94 according to this embodiment is formed as a hook type. Further, at an antinode position of the ultrasonic vibration or the vicinity of the position, the treatment portion 94 has a blade surface region 94a to which the ultrasonic vibration is transmitted in a state where the region is allowed to abut on the biological tissue, whereby it is possible to cut the biological tissue on which the region is allowed to abut.

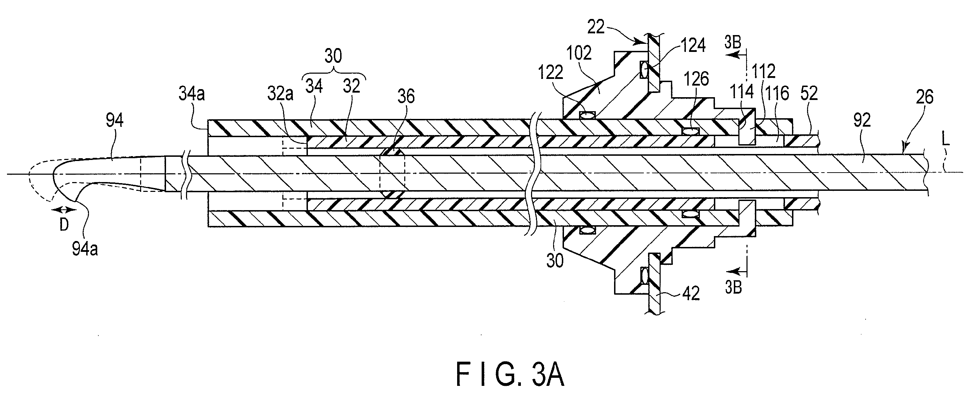

As shown in FIG. 3A, the tubular body 30 includes a tubular inner sheath 32 and a tubular outer sheath 34. The tubular body 30 is disposed on a distal end side of the holding portion 22, to cover an outer periphery of the probe main body 92 of the probe 26 in a state where the treatment portion 94 of the distal end portion of the probe 26 is exposed. The inner sheath 32 and the outer sheath 34 are concentrically arranged to the longitudinal axis L. The inner sheath 32 covers an outer peripheral surface of the probe 26. The outer sheath 34 covers an outer peripheral surface of the inner sheath 32. A proximal end of the inner sheath 32 is preferably formed integrally at a distal end of the inner case 52. It is to be noted that the outer sheath 34 has a length to expose the treatment portion 94 of the distal end portion of the probe 26. The inner sheath 32 has a length extending to a distal end of the probe 26 further from the node position generated on the most distal end side of the ultrasonic vibration in the probe. Consequently, a distal end 32a of the inner sheath 32 is positioned on a distal end side from the node position of the most distal end side of the ultrasonic vibration to be transmitted from the ultrasonic transducer unit 24 to the probe 26.

Between an inner peripheral surface of the inner sheath 32 of the tubular body 30 and the outer peripheral surface of the probe 26, there is interposed a sealing body (a first sealing member) 36 made of a rubber material or the like having heat resisting properties and shaped in the form of a ring. The sealing body 36 comes in contact closely with the inner peripheral surface of the inner sheath 32 of the tubular body 30 and the outer peripheral surface of the probe 26. In consequence, the sealing body 36 prevents penetration of a liquid from the sealing body (a sealing position) 36 along the longitudinal axis L into a proximal end side, when the liquid penetrates from a distal end side of the inner sheath 32 of the tubular body 30 into a space between the inner peripheral surface of the inner sheath and the outer peripheral surface of the probe 26. In addition, the sealing body 36 performs a function of a spacer that prevents the inner peripheral surface of the inner sheath 32 of the tubular body 30 from coming in contact with the outer peripheral surface of the probe 26. Consequently, in a state where the ultrasonic vibration is transmitted to the probe 26, the liquid or the inner sheath 32 is prevented from coming in contact with the probe main body 92 on the proximal end side from the sealing body 36. Further, when the ultrasonic vibration is transmitted to the probe 26, the transmission can be prevented from being obstructed, so that the ultrasonic vibration can suitably be transmitted to the treatment portion 94. In addition, a load can be prevented from being applied to the probe 26 as much as possible.

The sealing body 36 is disposed at a position corresponding to the node position of the ultrasonic vibration, when the vibration is transmitted from the ultrasonic transducer unit 24 to the probe 26. Although not shown in the drawings, the sealing bodies 36 are preferably disposed at respective node positions, when a length of the probe 26 is regulated so that the node positions of the vibration are present. When the node positions are present and one sealing body 36 is only disposed on the outer peripheral surface of the probe main body 92, the sealing body 36 is preferably disposed at a position corresponding to the most distal end node position, to prevent the penetration of the liquid into the inner sheath 32 as much as possible. Therefore, the distal end of the inner sheath 32 is positioned on the distal end side further from the position corresponding to the most distal end node position.

Further, the inner sheath 32 of the tubular body 30 moves together with the probe 26 and the sealing body 36 in accordance with the movement of the actuator 28. In other words, even when the actuator 28 operates, the inner sheath 32, the probe 26 and the sealing body 36 move in a state where a positional relation among them is substantially unchanged. At this time, the outer sheath 34 of the tubular body 30 does not move, and hence, the inner sheath 32 is relatively movable to the outer sheath 34 along the axial direction.

Further, an inner peripheral surface of the outer sheath 34 of the tubular body 30 may be slidable smoothly to the outer peripheral surface of the inner sheath 32 along the longitudinal axis L, or may be separated from the outer peripheral surface of the inner sheath. The outer sheath 34 of the tubular body 30 has, at its distal end, an opening 34a which is configured to change a projecting amount to the distal end when the ultrasonic transducer unit 24 and the probe 26 are moved along the longitudinal axis L by the actuator 28.

A core material of each of the inner sheath 32 and the outer sheath 34 of the tubular body 30 is made of a material such as a stainless alloy material having a rigidity, and each of an outer surface and an inner surface of the core material is preferably coated with a material such as PTFE having electric insulating properties.

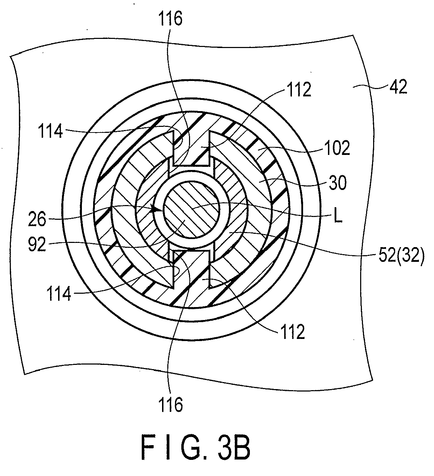

FIG. 3A and FIG. 3B show a coupling portion of the probe main body 92 and the proximal end of each of the inner sheath 32 and the outer sheath 34 of the tubular body 30 with the holding portion 22. As shown in FIG. 3A and FIG. 3B, a rotary operating knob 102 is attached to the inner case 52 so that the knob is rotatable in the periaxial direction of the longitudinal axis L of the tubular body 30. The rotary operating knob 102 is disposed on an outer peripheral side of the outer sheath 34 of the tubular body 30. The rotary operating knob 102 is integrally attached to the probe main body 92, the inner case 52 and the tubular body 30. Here, a coupling structure of the rotary operating knob 102, the probe main body 92, the inner case 52 and the tubular body 30 is described.

The rotary operating knob 102 has, in its proximal end portion, a pair of engaging claws 112 projected toward an inner side (the longitudinal axis L). The outer sheath 34 of the tubular body 30 has, in its proximal end portion, a pair of engaging holes 114 to be engaged with the engaging claws 112. When the engaging claws 112 of the rotary operating knob 102 are engaged with the engaging holes 114 of the outer sheath 34 of the tubular body 30, respectively, the outer sheath 34 of the tubular body 30 is attached to the rotary operating knob 102. The inner case 52 has, at its distal end portion, i.e., the inner sheath 32 has, at its proximal end portion, a pair of slide holes 116 in which the engaging claws 112 are relatively movable. Each of the slide holes 116 is shaped in the form of a long hole that is longer than a thickness of each of the engaging claws 112 along the axial direction of the longitudinal axis L of the probe main body 92. When the engaging claws 112 are inserted into the slide holes 116, the inner sheath 32, i.e., the inner case 52 is attached to the rotary operating knob 102. Consequently, when the inner sheath 32, i.e., the inner case 52 is attached to the rotary operating knob 102, the inner sheath 32 and the inner case 52 are relatively movable to the rotary operating knob 102 along the axial direction of the longitudinal axis L. In consequence, the inner sheath 32 of the tubular body 30, i.e., the inner case 52, the outer sheath 34 of the tubular body 30 and the rotary operating knob 102 can integrally be assembled. Further, when the rotary operating knob 102 is rotated in the periaxial direction of the longitudinal axis L of the probe 26, the outer sheath 34 and the inner sheath 32, i.e., the inner case 52 are integrally rotated in the periaxial direction of the longitudinal axis L together with the rotary operating knob 102. In conjunction with this operation of the inner case 52, the ultrasonic transducer unit 24 and the probe 26 are also rotated integrally with the outer sheath 34 and the inner sheath 32, i.e., the inner case 52 in the periaxial direction of the longitudinal axis L. It is to be noted that, in this embodiment, the rotary operating knob 102 is disposed, but does not necessarily have to be disposed, and the rotary operating knob 102 may suitably be disposed.

Further, O-rings 122 and 124 that maintain water tightness in the holding portion 22 are interposed between an outer peripheral surface of the outer sheath 34 and an inner peripheral surface of the rotary operating knob 102 and between the outer case 42 and a proximal end surface of the rotary operating knob 102, respectively.

In addition, an O-ring (a second sealing member) 126 is interposed between the outer peripheral surface of the inner sheath 32 and the inner peripheral surface of the outer sheath 34. The O-ring 126 is present at a position on the proximal end side from the sealing body 36 along the longitudinal axis L. The O-ring 126 maintains the water tightness when the inner sheath 32 moves to the outer sheath 34 along the axial direction of the longitudinal axis L, and maintains the water tightness when the inner sheath 32 rotates to the outer sheath 34 in the periaxial direction of the longitudinal axis L. Further, by an action (a frictional force) of the O-ring 122, the knob 102 and the outer sheath 34 are inhibited from mutually moving. By an action (a frictional force) of the O-ring 124, the knob 102 and the outer case 42 are inhibited from mutually moving. Further, by an action (a frictional force) of the O-ring 126, the inner sheath 32 and the outer sheath 34 are inhibited from mutually moving. Therefore, when the user does not operate the knob 102, the knob maintains its position. When the actuator 28 is not driven, the inner sheath 32 does not move to the outer sheath 34, and relative positions of the sheaths are maintained.

Additionally, in this embodiment, the O-rings 122 and 126 are disposed in the rotary operating knob 102. The rotary operating knob 102 is formed in a larger volume, has a higher rigidity and is harder to be deformed than another region. Consequently, the O-ring 122 is prevented from being shifted from a predetermined position on the outer peripheral surface of the outer sheath 34, and the O-ring 126 is prevented from being shifted from a predetermined position on the inner peripheral surface of the outer sheath 34. Therefore, the O-ring (the second sealing member) 126 enables the positional shift to the inner sheath 32 while maintaining a close contact state with the outer peripheral surface of the inner sheath 32, and the close contact state is maintained while preventing a positional shift to the outer sheath 34.

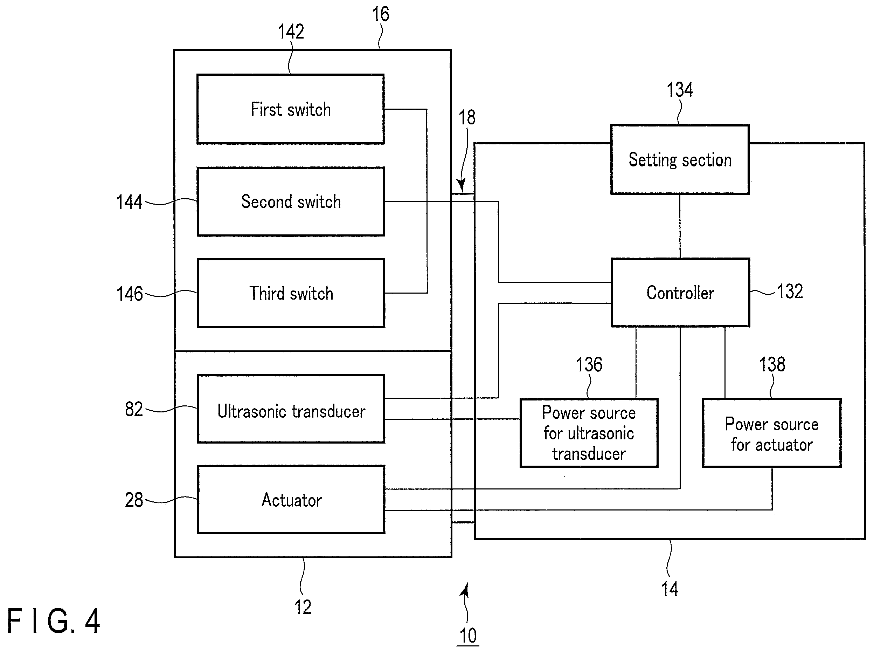

As shown in FIG. 4, the power source unit 14 includes a controller 132, the setting section 134, a power source 136 for the ultrasonic transducer as a first power source that supplies the power to the ultrasonic transducer 82, and a power source 138 for the actuator as a second power source that supplies the power to the actuator 28. The controller 132 is electrically connected to the setting section 134, the power source 136 for the ultrasonic transducer and the power source 138 for the actuator.

As shown in FIG. 1, the switch portion (the hand switch) 16 is disposed in the holding portion 22. The switch portion 16 is preferably formed in an outer peripheral surface of the holding portion 22 in the vicinity of a distal end portion of the holding portion. As shown in FIG. 1 and FIG. 4, here, the switch portion 16 includes a first switch 142, a second switch 144, and a third switch 146. It is to be noted that as shown in FIG. 8A of an after-mentioned third embodiment, the third switch 146 of the switch portion 16 is not necessarily required.

When the treatment device 12 is connected to the power source unit 14 via the cable 18, the power source 136 for the ultrasonic transducer is electrically connected to the ultrasonic transducer 82, and the power source 138 for the actuator is electrically connected to the actuator 28. In addition, when the treatment device 12 is connected to the power source unit 14 via the cable 18, the first switch 142, the second switch 144 and the third switch 146 are electrically connected to the controller 132.

The ultrasonic transducer 82 (the ultrasonic transducer unit 24) and the actuator 28 are controlled by the control section 132 on the basis of information set in the setting section 134 by the user. By use of the setting section 134, e.g., a touch panel, parameters concerning the treatment device 12, the power source unit 14 and the switch portion 16 can suitably be set.

In the setting section 134, a control parameter of the ultrasonic transducer 82 and a control parameter of the actuator 28 can suitably be set and stored. Hereinafter, there will be described one example of setting information to be input and stored in the setting section 134 by the user.

The user can suitably set the power to be supplied from the power source 136 for the ultrasonic transducer to the ultrasonic transducer 82 of the ultrasonic transducer unit 24, by the setting section 134. The ultrasonic transducer 82 is preferably set so that the treatment portion 94 of the probe 26 vibrates at a suitable amplitude irrespective of an impedance to be measured by the ultrasonic transducer 82. That is, the power source 136 for the ultrasonic transducer is controlled by the controller 132 so that the power (a voltage) is changed to maintain the amplitude in accordance with a measured value of the impedance.

The user can suitably set the power to be supplied from the power source 138 for the actuator to the actuator 28, by the setting section 134. The user can set control parameters such as a maximum moving amount (stroke) of the actuator 28 and a moving amount (speed) per unit time, by the setting section 134. The actuator 28 is preferably controlled by the control section 132 to straight move the treatment portion 94 of the probe 26 along the longitudinal axis L at a suitable stroke and a suitable speed irrespective of the impedance to be measured by the actuator 28. That is, the power source 138 for the actuator is controlled by the control section 132 so that the stroke is suitably maintained in accordance with the measured value of the impedance and so that the power (the voltage) is changed to suitably maintain the moving speed.

The user can assign functions of the first switch 142, the second switch 144 and the third switch 146 of the switch portion 16, by the setting section 134. The setting section 134 is preferably set so that the ultrasonic transducer 82 and the actuator 28 are not driven in a state where the pressed first to third switches 142, 144 and 146 are released.

The user sets the setting section 134 so that, for example, when the pressed state of the first switch 142 is maintained, the ultrasonic transducer 82 is driven to generate the ultrasonic vibration but the actuator 28 is not driven. The user sets the setting section 134 so that, for example, when the pressed states of the second and third switches 144 and 146 are maintained, the ultrasonic transducer 82 is driven to generate the ultrasonic vibration and the actuator 28 is driven. The user sets the setting section 134 so that, for example, when the pressed state of the second switch 144 is maintained, the stroke of the actuator 28 is smaller than that when the pressed state of the third switch 146 is maintained. In the state where the third switch 146 is pressed, the stroke becomes comparatively large, and hence, the setting section 134 is preferably set so that the moving speed is lower than that in a state where the second switch 144 is pressed.

Thus, on the basis of the state set by the setting section 134, the control section 132 applies the power from the power source 136 for the ultrasonic transducer to the ultrasonic transducer 82, and generates the ultrasonic vibration from the ultrasonic transducer 82. That is, the ultrasonic transducer 82 can be driven. Therefore, the ultrasonic vibration is transmitted from the ultrasonic transducer 82 through the horn 84 to the probe 26, i.e., the ultrasonic vibration is transmitted from the ultrasonic transducer unit 24 to the probe 26. In consequence, a treatment such as cutting of the biological tissue can be performed in the blade surface region 94a of the treatment portion 94 of the probe 26.

In addition, on the basis of the state set by the setting section 134, the controller 132 can apply the power from the power source 138 for the actuator to the actuator 28 and drive the actuator 28. Therefore, the linear actuating rod 62a of the linear motor 62 of the actuator 28 moves straight to the holding portion 22 along the longitudinal axis L, and the inner case 52 supporting the ultrasonic transducer unit 24 moves to the holding portion 22 along the longitudinal axis L. Here, in this embodiment, a position of the opening 34a of a distal end of the tubular body 30 (the outer sheath 34) to the holding portion 22 does not change, and hence, when the treatment portion 94 of the probe 26 moves along the longitudinal axis L, a distal end of the treatment portion 94 separates from and comes close to the distal end of the tubular body 30.

The user can set the setting section 134 so that, when the pressing is released, the generation of the ultrasonic vibration is simultaneously stopped in the state where the pressed first to third switches 142, 144 and 146 are released. The user can set the setting section 134 so that, for example, immediately after the generation of the ultrasonic vibration is stopped, the linear actuating rod 62a of the linear motor 62 of the actuator 28 is moved and returned to the most retracted position at a lower speed than the speed in the state where the second switch 144 or the third switch 146 is pressed.

It is to be noted that the user can set the setting section 134 so that, for example, in a state where the second switch 144 or the third switch 146 continues to be pressed, the vibration of the ultrasonic transducer 82 or the driving of the actuator 28 is stopped immediately after the actuator 28 moves as much as, e.g., one stroke, and in the state where the second switch 144 or the third switch 146 continues to be pressed, the vibration of the ultrasonic transducer 82 and the driving of the actuator 28 continue to be performed.

In addition, the user can set the treatment system 10 by the setting section 134 so that the first switch 142 preferentially operates when the first and second switches 142 and 144 are simultaneously pressed, the second switch 144 preferentially operates when the second and third switches 144 and 146 are simultaneously pressed, the first switch 142 preferentially operates when the first and third switches 142 and 146 are simultaneously pressed, and the first switch 142 preferentially operates when the first to third switches 142, 144 and 146 are simultaneously pressed.

Next, an operation of the treatment system 10 according to this embodiment will be described.

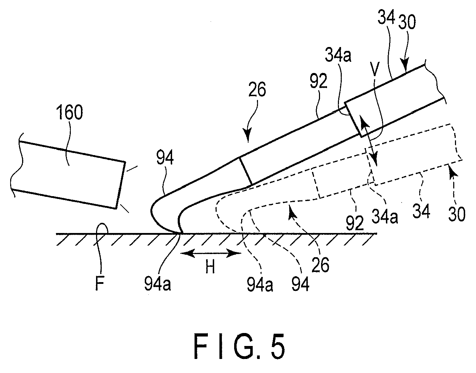

As shown in FIG. 5, when a surface F of a biological tissue such as a bone is cut, the cutting is performed while observing a portion of the biological tissue which is to be cut by an arthroscope 160 that is a type of endoscope. Here, for simplification of description, there is described a case where the treatment portion 94 of the probe 26 can continue to be observed by the arthroscope 160 without moving the arthroscope 160. That is, there is described a case where the treatment portion 94 of the probe 26 moves to come close to and separate from the arthroscope 160.

The user determines a range (a region) of the biological tissue to be cut in accordance with an observation result of the arthroscope 160 or the like. Further, the user suitably sets the speeds of the actuator 28 to be assigned to the second and third switches 144 and 146 (projecting and retracting speeds of the treatment portion 94 of the probe 26), and a control parameter such as the stroke on the basis of the observation result by the setting section 134 of the power source unit 14. The stroke to be assigned to the second switch 144 is set to be smaller than the stroke to be assigned to the third switch 146.

The user observes a direction of the probe 26 to the surface F of the biological tissue by the arthroscope 160 while holding the holding portion 22. The user rotates the rotary operating knob 102 to allow the blade surface region 94a of the treatment portion 94 of the probe 26 as needed, to abut on the surface F of the biological tissue.

When the pressed second switch 144 is maintained in this state, the ultrasonic transducer 82 is driven, and the ultrasonic vibration is generated from the ultrasonic transducer unit 24, i.e., the unit is driven. At this time, the blade surface region 94a of the treatment portion 94 enters in a depth direction (a direction to cut the biological tissue) from the surface F of the biological tissue by the action of the ultrasonic vibration. Simultaneously with or immediately after the generation of the ultrasonic vibration, the actuator 28 is driven. At this time, the inner sheath 32 moves forwardly and backwardly along the longitudinal axis L while maintaining a mutual close contact state of the sheaths against the frictional force of the O-ring 126 interposed between the outer peripheral surface of the inner sheath and the inner peripheral surface of the outer sheath 34. That is, the blade surface region 94a of the treatment portion 94 moves along the longitudinal axis L as much as a moving amount D that is the same amount as a moving amount D of the linear actuating rod 62a along the longitudinal axis L of the actuator 28. In consequence, the distal end of the treatment portion 94 of the probe 26 moves toward the arthroscope 160 while cutting the surface F of the biological tissue, and then the distal end separates from the arthroscope 160 while cutting the surface F of the biological tissue. That is, the blade surface region 94a moves in a region shown by reference sign H in FIG. 5. When the pressed second switch 144 continues to be maintained, the distal end of the treatment portion 94 of the probe 26 moves toward the arthroscope 160 again while cutting the surface F of the biological tissue, and then the distal end separates again from the arthroscope 160 while cutting the surface F of the biological tissue. That is, the blade surface region 94a reciprocates in the region shown by the reference sign H in FIG. 5. At this time, the outer sheath 34 and the O-ring 126 do not move, and the inner sheath 32 moves along the longitudinal axis L while the outer peripheral surface of the inner sheath is in contact closely with the O-ring 126. That is, a position of the inner sheath 32 is shifted to the outer sheath 34 by driving the actuator 28. On the other hand, the inner sheath 32 moves together with the probe 26. In consequence, even when the actuator 28 is driven, a position of the sealing body 36 does not shift to the probe 26 and the inner sheath 32. In other words, the actuator 28 moves the ultrasonic transducer unit 24, the probe 26, and the inner sheath 32 integrally to the outer sheath 34 along the longitudinal axis L at the stroke larger than the amplitude of the ultrasonic vibration to be transmitted to the treatment portion 94 of the probe 26.

The user needs to allow the blade surface region 94a of the treatment portion 94 of the probe 26 to abut on the surface F of the biological tissue. When a position of the holding portion 22 is simply fixed to the surface F of the biological tissue, for example, an angle of the blade surface region 94a changes, so that the cutting is hard to be stably performed. In consequence, the user moves the holding portion 22 substantially orthogonally to the longitudinal axis L as shown by reference sign V so that the angle of the blade surface region 94a of the treatment portion 94 of the probe 26 by the actuator 28 is suitably maintained. Here, it is not necessary to move the holding portion 22 along the longitudinal axis L (a substantially three-dimensional movement is not required), but if necessary, the holding portion may only be moved in a direction substantially orthogonal to the longitudinal axis L as shown by the reference sign V. The holding portion 22 is substantially two-dimensionally moved, whereby a region of the biological tissue along a substantially linearly set distance can be cut without manually moving the holding portion 22 in the direction along the longitudinal axis L.

The user cuts the biological tissue while confirming a cut state of the tissue by the arthroscope 160. Further, when it is confirmed that the biological tissue is cut by the ultrasonic vibration as much as a desirable amount, the pressed second switch 144 is released. Consequently, the driving of the ultrasonic transducer 82 is stopped. Immediately after the driving is stopped, the inner case 52, the ultrasonic transducer unit 24 and the probe 26 move to the holding portion 22 to be stopped so that the distal end of the treatment portion 94 comes closest to the distal end of the tubular body 30 by the operation of the actuator 28.

A treatment such as the cutting of the surface F of the biological tissue can be performed in this manner.

Here, when the pressed state of the first switch 142 is maintained, the ultrasonic transducer 82 is driven, but the actuator 28 is not driven. Consequently, the holding portion 22 is suitably moved in the direction along the longitudinal axis L and the abovementioned direction shown by the reference sign V, whereby the cutting of the biological tissue or the like can suitably be performed.

When the pressed state of the third switch 146 is maintained, the ultrasonic transducer 82 is driven, and the treatment portion 94 of the probe 26 can be moved at a stoke larger than that in a case where the pressed state of the second switch 144 is maintained by the actuator 28. Consequently, when the pressed state of the third switch 146 is maintained, a larger range of the biological tissue can be cut than the range in the case where the pressed state of the second switch 144 is maintained. That is, when the third switch 146 is pressed to be operated, an operation amount of the actuator 28 can be changed as compared with a case where the second switch 144 is pressed to be operated.

Here, there has been described the example where an initial position of the treatment portion 94 of the probe 26 to the tubular body 30 (a projecting position of the treatment portion 94 from the distal end of the tubular body 30 in a state where the ultrasonic transducer 82 and the actuator 28 are not driven) is set as a position of the treatment portion 94 brought closest to the distal end opening 34a of the tubular body 30. Needless to say, the setting section 134 can set the initial position of the treatment portion 94 of the probe 26 to the tubular body 30 as a position of the treatment portion 94 most separated from the distal end opening 34a of the tubular body 30. In addition, needless to say, the initial position can be set as a position between the closest position and the most separated position.

It is to be noted that the sealing body 36 is interposed between the outer peripheral surface of the probe 26 and the inner peripheral surface of the inner sheath 32, whereby the penetration of the liquid onto the proximal end side of the probe 26 from the position (the sealing position) of the sealing body 36 is prevented, and the outer peripheral surface of the probe 26 is separated from the inner peripheral surface of the inner sheath 32. In addition, a space between the outer peripheral surface of the inner sheath 32 and the inner peripheral surface of the outer sheath 34 is sealed with the O-ring (a sealing body) 126, and the liquid is prevented from penetrating into the outer peripheral surface of the probe 26 and the ultrasonic transducer unit 24. In consequence, the inner sheath 32 or the liquid is prevented from being brought into contact with a region other than the treatment portion 94 of the probe 26 to which the vibration is transmitted, and thus, a load is prevented from being applied to the region as much as possible.

As described above, the following things can be considered according to the treatment system 10 of this embodiment.

When the pressed state of the second switch 144 or the third switch 146 is maintained in the state where the holding portion 22 is held by the user, the ultrasonic transducer 82 and the actuator 28 are driven. Consequently, the treatment portion 94 can straight be moved in the state where the treatment portion 94 of the probe 26 is allowed to abut on the biological tissue. Therefore, according to the treatment device 12 of this embodiment, the treatment, e.g., the cutting of the biological tissue can straight be performed in the desirable range. At this time, the user hardly needs to move the position of the holding portion 22 in the axial direction of the longitudinal axis L, and may only maintain the state where the blade surface region 94a of the treatment portion 94 is allowed to abut on the surface F of the biological tissue, and hence, the user is hard to be tired. Therefore, according to this embodiment, there can be provided the treatment device 12 in which the user is hard to be tired when performing a treatment such as the cutting of the biological tissue, and the treatment system 10.

In addition, when the blade surface region 94a of the treatment portion 94 of the probe 26 is allowed to abut on the surface F of the biological tissue, the region can be directed in a desirable direction by rotating the rotary operating knob 102. Consequently, the holding portion 22 can continue to be held without changing the position of the switch portion 16 to the holding portion 22. In consequence, even when the biological tissue is a curved surface or the like, the state where the blade surface region 94a is allowed to abut on the biological tissue can easily be maintained, and the treatment can smoothly be performed.

The sealing body 36 is interposed between the outer peripheral surface of the probe 26 and the inner peripheral surface of the inner sheath 32, and hence, the penetration of the liquid onto the proximal end side of the probe 26 from the position (the sealing position) of the sealing body 36 can be prevented. In addition, the space between the outer peripheral surface of the inner sheath 32 and the inner peripheral surface of the outer sheath 34 is sealed with the O-ring (the sealing body) 126, so that the liquid can be prevented from penetrating into the outer peripheral surface of the probe 26 and the ultrasonic transducer unit 24. In consequence, the load can be prevented from being applied to the region other than the treatment portion 94 of the probe 26 to which the vibration is transmitted. Further, the power source 136 is controlled by the control section 132 so that the voltage is changed for the ultrasonic transducer unit 24 to maintain a constant amplitude, but the change of the voltage in this case can be inhibited.

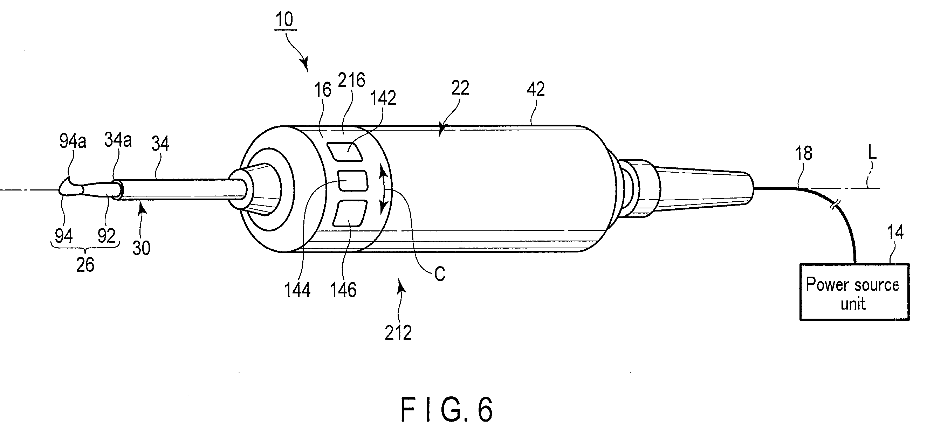

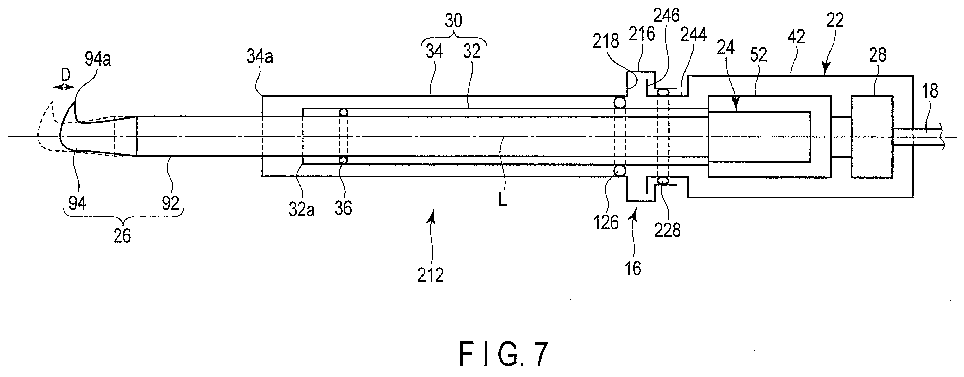

Next, a second embodiment will be described with reference to FIG. 6 and FIG. 7. This embodiment is a modification of the first embodiment, and the same members or members having the same functions as in members described in the first embodiment are denoted with the same reference signs as many as possible to omit descriptions of the members.

As shown in FIG. 6, in a treatment device 212 according to this embodiment, there are removed a tubular body 30 and a rotary operating knob 102 integrated with the tubular body 30 to the treatment device 12 according to the first embodiment shown in FIG. 1. In consequence, a probe 26 according to this embodiment does not rotate to an outer case 42 of a holding portion 22.

Here, instead, a switch portion 16 is rotatably disposed to the outer case 42 of the holding portion 22 in a periaxial direction of a longitudinal axis (a central axis) L. The switch portion 16 includes a ring body 216 rotatable to the outer case 42 in a peripheral direction C that is the periaxial direction of the longitudinal axis L. In consequence, first to third switches 142, 144 and 146 are disposed in the ring body 216.

As shown in FIG. 7, at a distal end of the outer case 42, a tubular portion 244 projecting to the longitudinal axis L outwardly in a radial direction is formed. An outer flange 246 is formed at a distal end of the tubular portion 244. In the ring body 216, a fitting groove 218 is formed into which the outer flange 246 is fitted. Consequently, the ring body 216 is rotatable to the outer case 42 in the periaxial direction of the longitudinal axis L. Thus, when the ring body 216 rotates to the outer case 42 in the periaxial direction of the longitudinal axis L, the first to third switches 142, 144 and 146 also rotate together.

To the ring body 216, a proximal end of an outer sheath 34 of the tubular body 30 is fixed. In consequence, when the ring body 216 rotates to the outer case 42 in the periaxial direction of the longitudinal axis L, the outer sheath 34 also rotates together with the first to third switches 142, 144 and 146.

Here, an O-ring (a second sealing member) 126 is interposed between an outer peripheral surface of an inner sheath 32 and an inner peripheral surface of the outer sheath 34, and an O-ring (a second sealing member) 228 is interposed between the outer flange 246 of the distal end of the outer case 42 and the fitting groove 218 of an inner peripheral surface of the ring body 216. In consequence, a liquid can be prevented from penetrating into the outer case 42 through a space between the outer peripheral surface of the inner sheath 32 and the inner peripheral surface of the outer sheath 34 and a space between the fitting groove 218 of the ring body 216 and the outer flange 246.

An operation of the treatment device 212 according to this embodiment will briefly be described.

A user directs a blade surface region 94a of a treatment portion 94 of the probe 26 to a surface F of a biological tissue while holding the holding portion 22. When the first to third switches 142, 144 and 146 of the switch portion 16 are present at positions hard to be operated, the switch portion 16 is rotated to the holding portion 22 along the peripheral direction C, and the first to third switches 142, 144 and 146 are maintained at positions easy to be operated by the user.

Further, when an actuator 28 is driven, the holding portion 22 and the ring body 216 are held. At this time, the outer sheath 34 and the O-ring 126 do not move, but the inner sheath moves along the longitudinal axis L against a frictional force while the outer peripheral surface of the inner sheath 32 is in contact closely with the O-ring 126. That is, a position of the inner sheath 32 is shifted to the outer sheath 34 by driving the actuator 28. On the other hand, the inner sheath 32 moves together with the probe 26. In consequence, even when the actuator 28 is driven, a position of a sealing body 36 is not shifted to the probe 26 and the inner sheath 32.

It is to be noted that, when the blade surface region 94a of the treatment portion 94 of the probe 26 is directed in a suitable direction, the holding portion 22 itself is rotated, and hence, a cable 18 needs to be rotated together. However, when a softer material having a high flexibility is selected as a material of the cable 18 and concerning a signal line in the cable, an influence on the user maintaining a position of the holding portion 22 can almost be ignored.

In consequence, the treatment device 212 according to this embodiment can be used substantially similarly to the treatment device 12 described in the first embodiment. That is, an operation of cutting, for example, a bone or a cartilate can be performed while preventing a liquid or an inner peripheral surface of the inner sheath 32 from being adhered to the probe 26 as much as possible and preventing a load due to the adhering of the liquid to the probe 26 to which an ultrasonic vibration is transmitted.

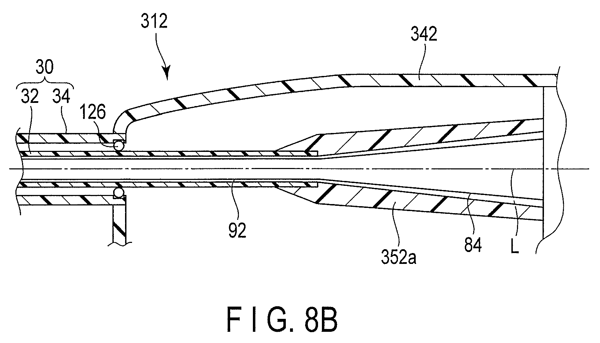

Next, a third embodiment will be described with reference to FIG. 8A and FIG. 8B. This embodiment is a modification of the first and second embodiments, and the same members or members having the same functions as in members described in the first and second embodiments are denoted with the same reference signs as many as possible to omit descriptions of the members.

As shown in FIG. 8A, as a treatment device 312 according to this embodiment, differently from the treatment device 12 described in the first embodiment and the treatment device 212 described in the second embodiment, there is described an example where a switch portion 16 is not disposed in a holding portion 22, and a foot switch 16a connected to a power source unit 14 is used as the switch portion 16. Here, the foot switch 16a has first and second switches 142 and 144 to which a function similar to that of the switch portion 16 described in the first and second embodiments is assigned. That is, the number of the switches may be three or two. However, there are preferably present a switch that does not drive an actuator 28 but drives an ultrasonic transducer 82 and a switch that drives the actuator 28 as well as the ultrasonic transducer 82.

The holding portion 22 includes a tubular body 342 in which the transducer unit 24 and the probe 26 are disposed at positions where a longitudinal axis L passes and the actuator 28 is disposed on an inner side. The transducer unit 24 and the probe 26 are movable to the tubular body 342 of the holding portion 22 along the longitudinal axis L, and the actuator 28 is fixed to the tubular body 342 of the holding portion 22. The actuator 28 of the treatment device 312 according to this embodiment is fixed to the tubular body 342 of the holding portion 22 at a position disposed away from the longitudinal axis L. That is, here, the arrangement of the actuator 28 is different from that of the first and second embodiments.

As shown in FIG. 8B, a proximal end of an outer sheath 34 of a tubular body 30 of this embodiment is fixed to a distal end of the tubular body 342 of the holding portion 22. A proximal end of an inner sheath 32 is fixed to an inner peripheral surface of a distal end of a tubular body 352a of an after-mentioned cover 352. Consequently, the inner sheath 32 moves together with the ultrasonic transducer unit 24 and the probe 26 by an actuation of the actuator 28, but the outer sheath 34 does not move together with the ultrasonic transducer unit 24 and the probe 26.

It is to be noted that as shown in FIG. 8A, there is disposed a sealing body 36 that seals a space between an inner peripheral surface of the inner sheath 32 and an outer peripheral surface of the probe 26 and prevents a liquid from penetrating from a sealing position to the outer peripheral surface of the probe 26 along the longitudinal axis L on a proximal end side. As shown in FIG. 8B, there is disposed an O-ring 126 that seals a space between an outer peripheral surface of the inner sheath 32 and an inner peripheral surface of the outer sheath 34 and prevents the liquid from penetrating into the outer peripheral surface of the probe 26 and the ultrasonic transducer unit 24. The O-ring 126 is disposed on an inner side of a distal end portion of the tubular body 342. The tubular body 342 is formed in a larger volume, has a higher rigidity and is harder to be deformed than another region. In consequence, even when the inner sheath 32 is moved to the outer sheath 34 along the longitudinal axis L, the O-ring 126 is prevented from being shifted from a predetermined position on the inner peripheral surface of the outer sheath 34.

In the first and second embodiments, there has been described the case where one cable 18 that supplies a power to the ultrasonic transducer unit 24 and the actuator 28 and further transmits a signal to the first and second switches 142 and 144 and receives a signal from the switches is extended from a proximal end portion of an outer case 42 of the holding portion 22. In this embodiment, a cable 18a for the transducer is extended from the ultrasonic transducer unit 24 to be attachably and detachably connected to the power source unit 14. In addition, a cable 18b for the actuator is extended from the actuator 28 to be attachably and detachably connected to the power source unit 14. Here, the foot switch 16a is connected to the power source unit 14, and hence, the signals of the first and second switches 142 and 144 do not have to be transmitted or received from the treatment device 312 to the power source unit 14.

The cables 18a and 18b are made of a material having a flexibility. As the material of the cables 18a and 18b and concerning a signal line in each cable, a softer material having a higher flexibility is preferably selected.

On an outer periphery of the ultrasonic transducer unit 24, the tubular cover (the cover for the transducer unit) 352 is disposed. A side surface of the cover 352 is supported movably in parallel with the longitudinal axis L by after-mentioned sliders 362a and 362b of the actuator 28 fixed to the holding portion 22.

Here, as the actuator 28 according to this embodiment, a linear motor may be used as described in the first embodiment, and a rotary motor including a ball screw attached to a rotary shaft may be used. Here, the actuator 28 has the plural sliders 362a and 362b (two sliders are described here) that perform a linear motion into which a rotary motion of the motor is converted by an operation of the ball screw.

The ultrasonic transducer unit 24 and the probe 26 are supported by the cover 352. The cover 352 supports the ultrasonic transducer unit 24 similarly to the inner case 52 described in the first embodiment. It is to be noted that the cover 352 has the distal end tube 352a that covers an outer periphery of a horn 84 at a distal end of the cover 352. The distal end tube 352a is formed to be tapered toward a distal end side while maintaining a state where an inner peripheral surface of the distal end tube 352a is separated from the horn 84. The proximal end of the inner sheath 32 is integrally and water-tightly fixed to the distal end of the tubular body 352a.

When the actuator 28 is driven, the cover 352 moves to the holding portion 22 along the longitudinal axis L. At this time, the ultrasonic transducer unit 24 and the probe 26 also move to the holding portion 22 along the longitudinal axis L together with the cover 352. Specifically, the cover 352 in which the probe 26 and the ultrasonic transducer unit 24 are supported linearly performs a one-way motion or a reciprocating motion once or plural times between a broken line position and a solid line position. At this time, the outer sheath 34 and the O-ring 126 do not move, and the inner sheath 32 moves along the longitudinal axis L together with the probe 26 while the outer peripheral surface of the inner sheath is in contact closely with the O-ring 126.

That is, when the second switch 144 of the foot switch 16a is pressed, the treatment device 312 can be used by driving the actuator 28 in the same manner as in the treatment device 12 described in the first embodiment. It is to be noted that when the first switch 142 of the foot switch 16a is pressed, needless to say, the treatment device 312 can be used in the same manner as in the treatment device 12 described in the first embodiment.

Here, when a direction of a blade surface region 94a of a treatment portion 94 of the probe 26 is moved in a peripheral direction to be opposed to a surface F of a biological tissue, the tubular body 342 of the holding portion 22 is rotated in a periaxial direction of the longitudinal axis L. At this time, as described in the second embodiment, the cables 18a and 18b have a flexibility, and hence, when a softer material having a higher flexibility is selected as a material of the cables 18a and 18b and concerning a signal line in each cable, an influence on a user maintaining a position of the holding portion 22 can almost be ignored.

In this embodiment, the example where the foot switch 16a is used has been described, but the switch portion 16 as a hand switch described in the first and second embodiments is also preferably used. In particular, the switch portion 16 having the ring body 216 described in the second embodiment is also preferably used. At this time, the longitudinal axis L of the ultrasonic transducer unit 24 and the probe 26 does not match a central axis of the tubular body 342 of the holding portion 22 (a central axis of the ring body 216) in a certain case, but the ring body 216 may only rotate in a periaxial direction of the central axis of the tubular body 342 of the holding portion 22 (the central axis of the ring body 216).

Additionally, this embodiment has been described on the assumption that an arrangement of the actuator 28 is different from that of the actuator 28 described in the first and second embodiments, but needless to say, the actuator 28 described in the first and second embodiments may be used as it is.

Next, a fourth embodiment will be described with reference to FIG. 9. This embodiment is a modification of the first to third embodiments, and the same members or members having the same functions as in members described in the first to third embodiments are denoted with the same reference signs as many as possible to omit descriptions of the members. It is to be noted that this embodiment is an example where a foot switch 16a has first to third switches 142, 144 and 146.

A treatment device 412 according to this embodiment includes a tubular body 430 that is different from the tubular body 30 of each of the treatment devices 12 and 312 described in the first and third embodiments. The tubular body 430 includes an inner sheath 32 and an outer sheath 434.

As shown in FIG. 9, a distal end of the outer sheath 434 of the tubular body 430 is closed, and an opening 434a is formed in a side surface of the outer sheath in the vicinity of a distal end portion of the outer sheath 434. A blade surface region 94a of a treatment portion 94 of a distal end portion of a probe 26 is positioned on a slightly outer side of the tubular body 430 than the opening 434a. That is, in a side surface of a distal end portion of the tubular body 430, the tubular body includes the opening 434a through which the treatment portion 94 of the distal end portion of the probe 26 is exposed. The opening 434a is preferably formed to be longer in a direction parallel to a longitudinal axis L than in a peripheral direction.

It is to be noted that an actuator 28 according to this embodiment is set and controlled by a setting section 134 so that the treatment portion 94 is movable and does not abut on a distal edge portion 435a and a proximal edge portion 435b of the opening 434a of the tubular body 430.

The treatment device 412 according to this embodiment can be used in the same manner as in the treatment device 312 described in the third embodiment. That is, when the second switch 144 or the third switch 146 of the foot switch 16a is pressed, the treatment device 412 can be used in the same manner as in the treatment device 12 described in the first embodiment. It is to be noted that when the first switch 142 of the foot switch 16a is pressed, needless to say, the treatment device 412 can be used in the same manner as in the treatment device 12 described in the first embodiment.

The distal end of the tubular body 430 is closed, and hence, even when a distal end of the tubular body 430 is allowed to abut on a biological tissue such as a bone in a state where an ultrasonic vibration is transmitted to the probe 26, the treatment portion 94 does not abut.

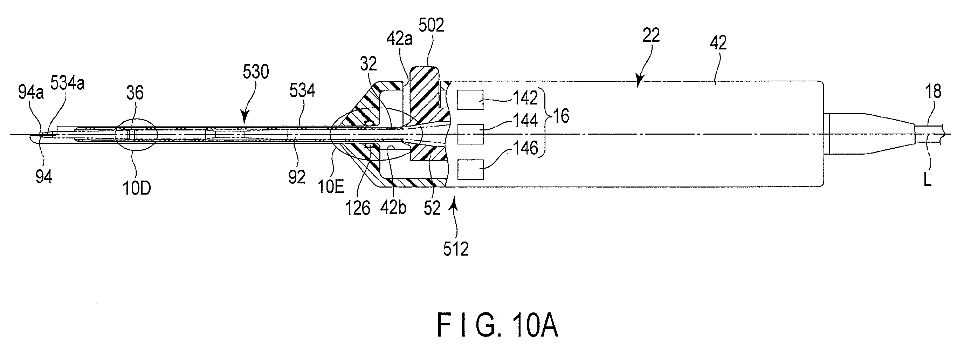

Next, a fifth embodiment will be described with reference to FIG. 10A to FIG. 10E. This embodiment is a modification of the first to fourth embodiments, and the same members or members having the same functions as in members described in the first to fourth embodiments are denoted with the same reference signs as many as possible to omit descriptions of the members. It is to be noted that this embodiment is described on the assumption that a switch portion 16 as a hand switch is disposed in a holding portion 22.

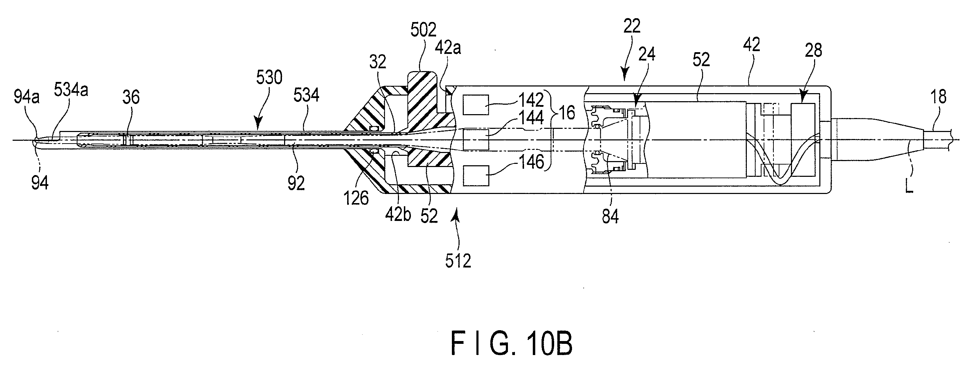

As shown in FIG. 10A and FIG. 10B, a treatment device 512 according to this embodiment includes a tubular body 530 that is different from the tubular body 30 of each of the treatment devices 12 and 312 described in the first and third embodiments and the tubular body 430 of the treatment device 412 described in the fourth embodiment. The tubular body 530 includes an inner sheath 32 and an outer sheath 534. A distal end portion of the outer sheath 534 is capable of performing a treatment with an exposed blade surface region 94a, but the distal end portion protects a distal end of a treatment portion 94 and a back surface side to the blade surface region 94a.

As shown in FIG. 10A to FIG. 10C, in the treatment device 512 according to this embodiment, an inner case 52 in which a transducer unit 24 is disposed, a probe main body 92 and an inner sheath 32 of the tubular body 530 in which the probe main body 92 is inserted can be moved in a predetermined range along a longitudinal axis L by an actuator 28 disposed in an outer case 42 in the same manner as described in the first embodiment.

Further, differently from the first embodiment, the treatment device 512 includes an opening 42a formed in the vicinity of a distal end portion of the outer case 42, and a lever (a rotary body) 502 rotatable in a periaxial direction of the longitudinal axis L in the predetermined range. The lever 502 is integrated with the inner case 52 and the inner sheath 32. Consequently, when the lever 502 is rotated in the periaxial direction of the longitudinal axis L, the ultrasonic transducer unit 24 and a probe 26 also rotate together with the inner case 52 and the inner sheath 32. It is to be noted that the lever 502 is movable in the opening 42a in parallel with the longitudinal axis L by the actuator 28. This movement of the lever 502 along the longitudinal axis L appears as a movement of the treatment portion 94 shown from FIG. 10A to FIG. 10B and/or a movement shown from FIG. 10B to FIG. 10A.

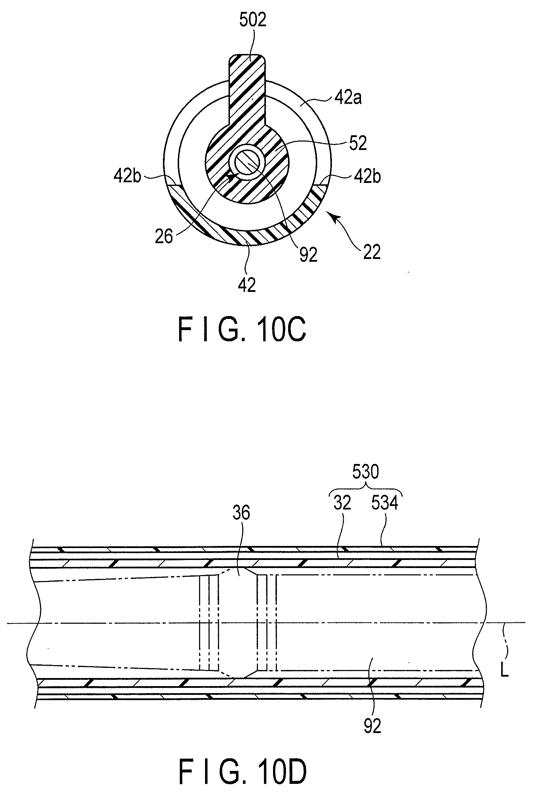

The outer case 42 has a pair of end faces 42b that define a rotating range of the lever 502. Consequently, the lever 502 rotates in the predetermined range. In FIG. 10C, the lever 502 is rotatable as much as about 180º.

Here, a proximal end of the outer sheath 534 of the tubular body 530 is fixed to a distal end of the outer case 42. The distal end portion of the outer sheath 534 has an opening 534a in a part of a distal end of the outer sheath 534 to a side surface thereof. Consequently, when the lever 502 is rotated in the periaxial direction of the longitudinal axis L, the treatment portion 94 of the probe 26 can be rotated in the periaxial direction of the longitudinal axis L of the treatment portion to expose and hide the blade surface region 94a while protecting the distal end of the treatment portion 94. In consequence, by this rotation of the lever 502, the lever 502 can be directed in a suitable direction to the opening 534a of the distal end portion of the outer sheath 534 of the blade surface region 94a of the treatment portion 94.

As shown in FIG. 10D, a sealing body 36 as a first sealing member is interposed between an outer peripheral surface of the probe main body 92 of the probe 26 and an inner peripheral surface of the inner sheath 32. Consequently, a liquid is prevented from penetrating from the treatment portion 94 of the probe 26 along the inner peripheral surface of the inner sheath 32 and the outer peripheral surface of the probe main body 92 onto a proximal end side of the sealing body 36, and the inner peripheral surface of the inner sheath 32 is prevented from being brought into contact with the outer peripheral surface of the probe main body 92.

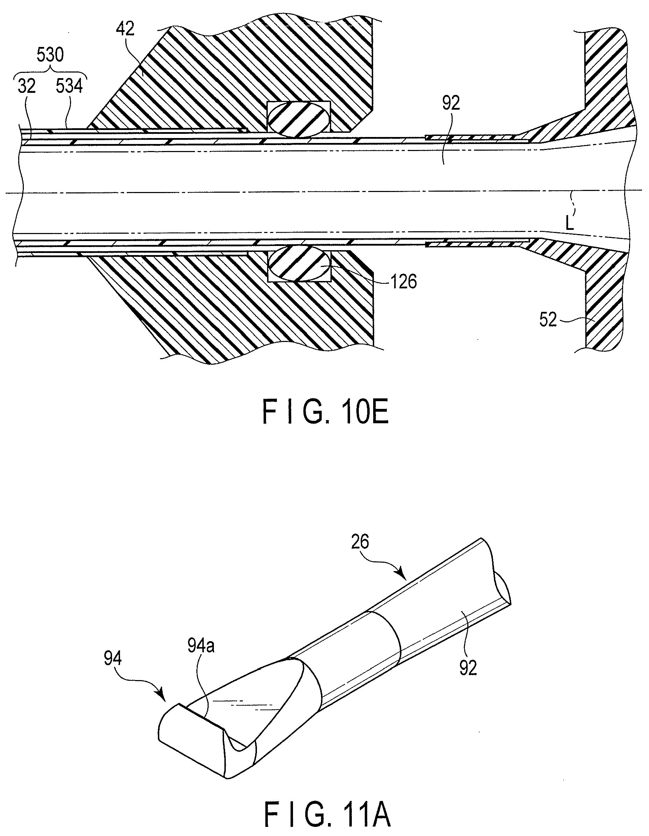

As shown in FIG. 10E, an O-ring 126 as a second sealing member is interposed between an outer peripheral surface of the inner sheath 32 and an inner peripheral surface of the outer case 42. That is, the O-ring 126 according to this embodiment does not directly come in contact with an inner peripheral surface of the outer sheath 534. Even when the O-ring 126 is formed in this manner, the O-ring can seal a space between the outer peripheral surface of the inner sheath 32 and the inner peripheral surface of the outer sheath 534. The O-ring 126 is disposed on an inner side of the distal end portion of the outer case 42. The outer case 42 is formed in a larger volume, has a higher rigidity, and is harder to be deformed than another region. In consequence, the O-ring 126 here is disposed in the holding portion 22, a position of the O-ring can be shifted to the inner sheath 32 while maintaining a close contact state with the outer peripheral surface of the inner sheath 32, and the close contact state is maintained while preventing the positional shift of the O-ring to the holding portion 22.

Further, the actuator 28 is operated by suitably operating the switch portion 16 shown in FIG. 10A and FIG. 10B, whereby the distal end of the treatment portion 94 can be moved between a position shown in FIG. 10A and a position shown in FIG. 10B.

Further, when the actuator 28 is driven, the outer case 42, the outer sheath 534 and the O-ring 126 do not move, but the inner sheath 32 moves along the longitudinal axis L while the outer peripheral surface of the inner sheath is in contact closely with the O-ring 126. That is, a position of the outer sheath 534 is shifted to the inner sheath 32 by driving the actuator 28. The inner sheath 32 moves together with the probe 26. Consequently, a position of the sealing body 36 is not shifted to the probe 26 and the inner sheath 32 even by driving the actuator 28. In consequence, a load is prevented from being applied to the proximal end side of the sealing body 36 in the probe 26 as much as possible.

Further, for example, a predetermined range of a bone, a cartilage or the like can be cut without moving the holding portion 22. It is to be noted that at this time, the lever 502 also moves along the longitudinal axis L between the openings 42a of the outer case 42.

It is to be noted that in the abovementioned first to fifth embodiments, the example where the motor is used as the actuator 28 has been described, but a solenoid may be interposed between the outer peripheral surface of the inner case 52 or the cover 352 and the inner peripheral surface of the outer case 42 or the tubular body 342, and the inner case 52 or the cover 352 may be moved to the outer case 42 or the tubular body 342 in the axial direction of the longitudinal axis L by a magnetic force.

Next, a modification of the treatment portion 94 of the probe 26 described in the first to fifth embodiments will be described.

It has been described that the treatment portion 94 of the first to fifth embodiments is the hook type.

As shown in FIG. 11A, the treatment portion 94 may be formed as a rake type. A blade surface region 94a of the rake type of treatment portion 94 is formed in a suitable range in a direction orthogonal to a longitudinal axis L. The blade surface region 94a is allowed to abut on a surface F of a biological tissue, so that a treatment can be performed by suitably using the treatment devices 12, 212, 312, and 412 described in the first to fifth embodiments.

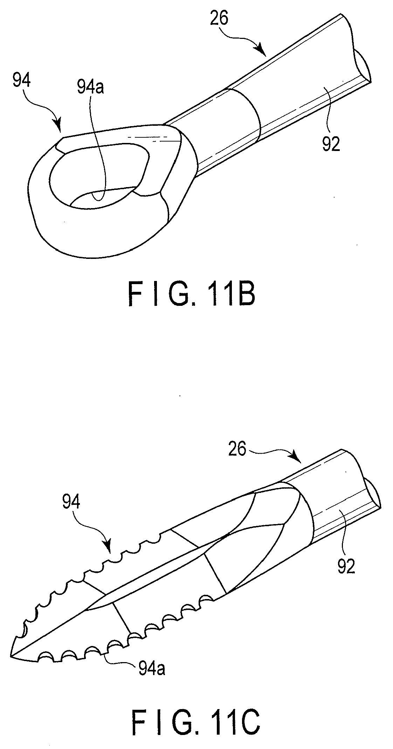

As shown in FIG. 11B, the treatment portion 94 may be formed as a curette type. A blade surface region 94a of the curette type of treatment portion 94 is formed in a ring shape. The blade surface region 94a is allowed to abut on a surface F of a biological tissue, whereby a treatment can be performed by suitably using the treatment devices 12, 212 and 312 described in the first to third embodiments. In addition, an opening 34a is regulated, whereby the treatment can be performed also by suitably using the treatment device 412 described in the fourth and fifth embodiments.

The treatment portions 94 shown in FIG. 11A and FIG. 11B are suitable for a treatment of cutting a joint cartilate in the in the same manner as in the treatment portion 94 described in the first to fifth embodiments.

As shown in FIG. 11C, the treatment portion 94 may be formed as a blade type. A blade surface region 94a of the blade type of treatment portion 94 is substantially formed into a U-shape. The blade surface region 94a is allowed to abut on a surface F of a biological tissue, whereby a treatment can be performed by suitably using the treatment devices 12, 212, 312, 412 and 512 described in the first to fifth embodiments. The treatment portion 94 shown in FIG. 11C is suitable for a treatment of cutting and removing an articular capsule or an articular lip.

In addition, treatment portions 94 having various shapes are usable.

Hitherto, several embodiments have specifically been described with reference to the drawings, but this invention is not limited to the abovementioned embodiments, and includes all implementations to be performed without departing from the gist of the invention.

### Reference Signs List

12 ... treatment device, 16 ... switch portion, 18 ... cable, 22 ... holding portion, 24 ... ultrasonic transducer unit, 26 ... probe, 28 ... actuator, 30 ... tubular body, 32 ... inner sheath, 34 ... outer sheath, 36 ... sealing body, 42 ... outer case, 52 ... inner case, 62a ... linear actuating rod, 72 ... bearing, 74 ... bearing, 82 ... ultrasonic transducer, 84 ... horn, 92 ... probe main body, 94 ... treatment portion, 94a ... blade surface region, and 126 ... O-ring.

## Claims

1. A treatment device for use in treating a biological tissue, comprising:
a holding portion to be held by a user;
an ultrasonic transducer unit that generates an ultrasonic vibration in accordance with a supply of a power;
a probe which defines a longitudinal axis, is configured to transmit the ultrasonic vibration generated by the ultrasonic transducer unit, and has a treatment portion configured to treat the biological tissue by an action of the ultrasonic vibration transmitted to a distal end portion of the probe;
an inner sheath that covers an outer peripheral surface of the probe;
a first sealing member that is interposed between an inner peripheral surface of the inner sheath and the outer peripheral surface of the probe, and comes in contact closely with the inner peripheral surface of the inner sheath and the outer peripheral surface of the probe to prevent penetration of a liquid;
an outer sheath that covers an outer peripheral surface of the inner sheath;
a second sealing member that is interposed between the outer peripheral surface of the inner sheath and an inner peripheral surface of the outer sheath, and comes in contact closely with the outer peripheral surface of the inner sheath and the inner peripheral surface of the outer sheath to prevent the penetration of the liquid; and
an actuator that moves the ultrasonic transducer unit, the probe and the inner sheath integrally to the outer sheath along the longitudinal axis at a stroke larger than an amplitude of the ultrasonic vibration to be transmitted to the treatment portion of the probe.

2. The treatment device according to claim 1, wherein
a distal end of the inner sheath is positioned on a distal end side from a node position of the most distal end side of the ultrasonic vibration transmitted to the probe, and
the first sealing member is positioned at the node position of the most distal end side of the ultrasonic vibration transmitted to the probe.

3. The treatment device according to claim 1, wherein the second sealing member enables a positional shift of the second sealing member to the inner sheath while maintaining a close contact state with the outer peripheral surface of the inner sheath, and a positional shift of the second sealing member to the outer sheath is prevented.

4. The treatment device according to claim 1, wherein the second sealing member is disposed in the holding portion, and enables a positional shift of the second sealing member to the inner sheath while maintaining a close contact state with the outer peripheral surface of the inner sheath, and a positional shift of the second sealing member to the holding portion is prevented.

5. The treatment device according to claim 1, wherein the first sealing member is disposed at a position that becomes a node of the ultrasonic vibration, when the vibration generated by the ultrasonic transducer unit is transmitted to the probe.

6. The treatment device according to claim 1, wherein the second sealing member is present at a position on a proximal end side from the first sealing member along the longitudinal axis.

7. The treatment device according to claim 1, comprising a knob that rotates the ultrasonic transducer unit, the probe, the inner sheath, and the outer sheath to the holding portion in a periaxial direction of the longitudinal axis of the probe.

8. The treatment device according to claim 1, further comprising a cover that is interposed between the holding portion and the ultrasonic transducer unit, supports the ultrasonic transducer unit at a node position of the ultrasonic vibration, and is moved by the actuator to move the ultrasonic transducer unit and the probe along the longitudinal axis of the probe at the stroke larger than the amplitude of the ultrasonic vibration to be transmitted to the treatment portion of the probe.

9. The treatment device according to claim 8, wherein the outer sheath includes, at its distal end, an opening which is configured to change a projecting amount to the distal end when the ultrasonic transducer unit and the probe are moved along the longitudinal axis by the actuator.

10. The treatment device according to claim 8, wherein the outer sheath includes, in a side surface of its distal end portion, an opening through which the treatment portion of a distal end portion of the probe is exposed.

11. A treatment system comprising:
the treatment device according to claim 1; and
a controller that controls the actuator.

12. The treatment system according to claim 11, wherein
the controller is attachably and detachably connected to a switch portion, and
the switch portion includes:
a first switch that does not operate the actuator while the ultrasonic transducer unit generates the ultrasonic vibration; and
a second switch that operates the actuator while the ultrasonic transducer unit generates the ultrasonic vibration.

13. The treatment system according to claim 12, wherein the switch portion further includes a third switch that is able to operate the actuator while the ultrasonic transducer unit generates the ultrasonic vibration and that changes an operation amount of the actuator as compared with a case where the second switch is operated.

14. The treatment system according to claim 12, wherein the switch portion is disposed on the holding portion.

15. The treatment system according to claim 12, wherein the switch portion is disposed on an outer periphery of the holding portion to be rotatable to the holding portion in a periaxial direction of the longitudinal axis.
